# EUROPÄISCHE PATENTSCHRIFT

(11) **EP 0 518 907 B1**
(45) Veröffentlichungstag und Bekanntmachung des Hinweises auf die Patenterteilung: **14.12.1994**
(21) Anmeldenummer: 91905030.2
(22) Anmeldetag: 27.02.1991
(51) Int. Cl.: G01N 33/04, G01N 27/06

(54) **VERFAHREN ZUR ÜBERWACHUNG DER QUALITÄT BEI DER MILCHABGABE VON TIEREN**
PROCESS FOR MONITORING THE QUALITY OF MILK DURING MILKING OF ANIMALS
PROCEDE DE CONTROLE DE LA QUALITE DU LAIT PENDANT LA TRAITE D'ANIMAUX

(30) Priorität: 08.03.1990 DE 4007327
(43) Veröffentlichungstag der Anmeldung: 23.12.1992
(73) Patentinhaber: LANG APPARATEBAU GMBH, 83313 Siegsdorf (DE)
(72) Erfinder: WAGNER, Georg, F., D-8240 Berchtesgaden (DE)
(74) Vertreter: Wilk, Hans-Christof, Dr.
(86) Internationale Anmeldenummer: EP9100364
(87) Internationale Veröffentlichungsnummer: WO9114179

(56) Entgegenhaltungen:
- EP-A- 222 612
- US-A- 4 325 028
- US-A- 4 793 285

## Beschreibung

Die Erfindung betrifft ein Verfahren der im Oberbegriff des Patentanspruchs 1 angegebenen Art.

Es ist bekannt, daß die elektrische Leitfähigkeit der Milch von Kühen und anderen Tieren bei Entzündungen von Drüsen im Zitzenbereich einen gegenüber dem gesunden Zustand des Tieres veränderten Wert aufweist. Im Falle einer Entzündung liegt eine erhöhte elektrische Leitfähigkeit vor. Die frühzeitige Erkennung einer solchen Entzündung (Mastitis) ist bei Kühen von großer Wichtigkeit, weil geeignete Gegenmaßnahmen getroffen werden können, bevor die Entzündung sichtbar wird und weil andererseits die aus entzündeten Drüsen stammende Milch einen höhen Gehalt an schädlichen Keimen hat. Milch, die aus von Mastitis befallenen Zitzen stammt, unterscheidet sich von der Milch aus gesunden Zitzen durch erhöhte elektrische Leitfähigkeit.

Das Leitwertsignal, das von einer Leitfähigkeits-Meßzelle erzeugt wird, die im Strömungsweg der Milch einer Zitze angeordnet ist, ist ein stark verrauschtes Signal. Insbesondere bei unzureichender Milchabgabe entstehen starke Signalschwankungen, die keine verwertbare Leitfähigkeitsmessung erlauben. Eine andere Schwierigkeit besteht darin, daß die Leitfähigkeit der Milch Schwankungen unterworfen ist, die jahreszeitlich bedingt sind und die auch von anderen Faktoren, wie z. B. der Zusammensetzung des Futters, abhängen. Aufgrund der genannten Schwierigkeiten ist bisher eine Früherkennung von Zitzenerkrankungen anhand des elektrischen Leitwerts der abgegebenen Milch im on-line-Verfahren, also unmittelbar bei der Milchabnahme vom Tier, nur mit erheblichen Unsicherheiten möglich.

Ein derartiges Verfahren ist aus der US-A-3,989,009 bekannt. Hierbei wird Milch in Röhrchen durch eine Vorrichtung geführt, der ein Signalerzeuger zugeordnet ist. Die von dem Signalerzeuger abgegebenen Signale werden durch die unterschiedliche Leitfähigkeit der Milch in den einzelnen Röhrchen modifiziert. Der Vorrichtung sind Auswerteeinrichtungen für die Leitfähigkeitssignale nachgeschaltet.

Aus der EP-A-0 222 612 ist es für die Analyse von chromatographischen Daten bekannt, die bei Leitfähigkeitsmessungen erhaltenen Signale mit Hilfe eines Filters deutlicher hervortreten und besser unterscheidbar zu machen.

Ein Verfahren mit den im Oberbegriff des Patentanspruches 1 angegebenen Merkmalen ist weiterhin auch aus der US-A-4,325,028 bekannt. Hierbei wird aus den den einzelnen Zitzenbechern zugeordneten Leitfähigkeitssignalen das kleinste Leitfähigkeitssignal ermittelt und in Komperatorschaltung werden die Leitfähigkeitssignale der Milch der anderen Zitzenbecher mit dem kleinsten Leitfähigkeitssignal verglichen, wobei jeweils die Differenz gebildet wird. Die einzelnen Differenzen werden jeweils mit einem festgelegten oberen und unteren Grenzwert verglichen. Außerdem wird festgestellt, ob das kleinste Leitfähigkeitssignal über einem weiteren oberen Grenzwert liegt. Die Verarbeitung der Leitfähigkeitssignale erfolgt analog, ohne daß eine Frequenzfilterung vorgesehen wäre. Es werden auch nur diejenigen Leitfähigkeitssignale miteinander verglichen, die bei einem Melkvorgang gleichzeitig auftreten.

Die US-A-4,225,820 beschreibt ein Verfahren, bei dem die Leitfähigkeitssignale der Milch der einzelnen Zitzenbecher jeweils nur mit einem oberen Grenzwert verglichen werden. Eine Anzeigevorrichtung zeigt an, ob dieser Grenzwert von der Milch einer einzelnen Zitze überschritten wird.

Weiterhin ist aus der US-A-4,793,285 ein Verfahren zur Überwachung der Qualität bei der Milchabgabe von Tieren bekannt. Bei diesem Verfahren werden die erfaßten Leitfähigkeitsmeßsignale einem Analog-digital-Wandler zugeführt. Ein Vergleich der jeweils ermittelten, einzelnen Meßleitfähigkeitssignale ist aber nicht vorgesehen.

Der Erfindung liegt die Aufgabe zugrunde, ein Verfahren der im Oberbegriff des Patentanspruches 1 angegebenen Art zu schaffen, das eine sichere Erkennung von Drüsenentzündungen an einer Zitze unmittelbar bei der Milchabgabe ermöglicht.

Die Lösung dieser Aufgabe erfolgt erfindungsgemäß mit den im Patentanspruch 1 angegebenen Merkmalen.

Nach der Erfindung werden die Leitfähigkeitssignale der einzelnen Meßzellen, von denen jede einem Zitzenbecher zugeordnet ist, zunächst einer digitalen Filterung mit einem Signalprozessor unterzogen, wobei Signalfrequenzen unterdrückt werden, die über 0,2 Hz liegen. Ein analog arbeitendes Tiefpaßfilter mit einer derartig niedrigen Grenzfrequenz ist nicht oder nur mit großem Aufwand realisierbar. Zum Herausfiltern der höherfrequenten Störsignale wird daher der digitale Signalprozessor benutzt, der eine Spektralanalyse der ihm zugeführten elektrischen Signale durchführt, beispielsweise eine Fast-Fourier-Transformation. Auf diese Weise kann mit einfachen technischen Mitteln eine Tiefpaßfilterung bei sehr niedriger oberer Grenzfrequenz durchgeführt werden. Die obere Grenzfrequenz muß nicht notwendigerweise 0,2 Hz betragen, sondern sie kann auch tiefer liegen, beispielsweise bei 0,1 Hz.

Die auf diese Weise gefilterten Leitfähigkeitssignale, die den höherfrequenten Störanteil nicht mehr enthalten, werden von Meßzelle zu Meßzelle untereinander verglichen, so daß krankheitsbedingte Abweichungen einzelner Zitzen leicht festgestellt werden können. Vorzugsweise wird eine Zitze dann als krank erkannt, wenn das gefilterte Leitfähigkeitssignal der von ihr gelieferten Milch um einen festgelegten (einstellbaren) Prozentsatz über den Leitfähigkeitssignalen der von den übrigen Zitzen gelieferten Milch um einen festgelegten (einstellbaren) Prozentsatz über den Leitfähigkeitssignalen der von den übrigen Zitzen gelieferten Milch liegt. Um auch den Fall einzuschließen, daß mehrere Zitzen erkrankt sind, kann der Vergleich jeweils mit dem Leitwert der Milch erfolgen, die von derjenigen Zitze, deren Milch den geringsten Leitwert hat, geliefert wird.

Der Melkvorgang wird für jeden Zitzenbecher in Zeitintervalle aufgeteilt, die jeweils eine vorbestimmte Zeitdauer haben. Der gesamte Melkprozess besteht somit aus zahlreichen untereinander gleich langen Zeitintervallen, die laufend durchnumeriert werden. In jedem Zeitintervall wird ein charakteristischer Leitfähigkeitswert ermittelt, der ausgewertet und gespeichert wird. Der charakteristische Leitfähigkeitswert ist vorzugsweise der auswertbare Spitzenwert, der in dem betreffenden Intervall aufgetreten ist, jedoch kann auch ein anderer Wert, beispielsweise der Mittelwert dieses Intervalls, als charakteristischer Wert benutzt werden.

In den verschiedenen Phasen des Melkvorganges, der aus dem Vorgemelk, dem Hauptgemelk und dem Nachgemelk besteht, treten normalerweise unterschiedliches Leitwerte auf. Die Melkvorgänge der einzelnen Zitzen sind dadurch gegeneinander verschoben, daß die Zitzenbecher nacheinander an die Zitzen angesetzt werden. Dadurch, daß jedem Zitzenbecher eine eigenen Intervallfolge zugeordnet wird, können die einander innerhalb des Melkvorganges entsprechenden Intervalle miteinander verglichen werden, obwohl diese Intervalle gegeneinander zeitversetzt sind. Verglichen werden jeweils die charakteristischen Leitfähigkeitswerte von Intervallen, deren Nummern gleich sind.

Jeder der Melkvorgänge, die morgens und abends, Tag für Tag, durchgeführt werden, kann sich über mehrere Tage hinweg von anderen Melkvorgängen unterscheiden. Für eine Analyse des Gesundheitszustandes des Euters des Tieres kann es zweckmäßig sein, die Melkvorgänge über eine längere Zeit hinweg miteinander zu vergleichen, um normale Trends oder abnormale Erhöhungen des Milch-Leitwertes zu erkennen. In der Regel sind Differenzen im Leitwert von 1000 µS/cm innerhalb von 3 Tagen auffällig.

Das erfindungsgemäße Verfahren erlaubt es, die charakteristischen Leitfähigkeitswerte der zahlreichen Intervalle eines Melkvorganges abzuspeichern und mit den charakteristischen Leitfähigkeitswerten mehrerer vorhergehender Melkvorgänge zu vergleichen. Der Verdacht einer subklinischen Melkvorgänge ist immer dann anzunehmen, wenn die Differenzleitfähigkeit innerhalb weniger Melkvorgänge um einige hundert Mikro-Siemens nach oben springt. Dabei ist die absolute Höhe des Leitfähigkeitswertes unbeachtlich. Der klassische Fall der subklinischen Mastitis besteht darin, daß aus einer Zitze Milch mit einem Leitwert abgegeben wird, der um 1 000 oder 2 000 µS höher ist als derjeniger der Milch der anderen drei Zitzen.

Wenn die Leitfähigkeit der Milch der drei gesunden Zitzen innerhalb eines Abweichungsbereichs von ca. 200 bis 400 µS liegt, so kann dies als normal angenommen werden. Die Leitfähigkeit einer erkrankten Zitze weicht demgegenüber regelmäßig um mehr als 1 000 µS nach oben ab.

Gegen Ende des Melkvorgangs sinkt die Leitfähigkeit der Milch wegen des steigenden Fettgehalts beim Nachgemelk (Stripping) ab. Durch Vergleich der Leitfähigkeitsmeßkurve des gesamten Melkvorgangs mit einer Referenzkurve kann ein abnormaler Verlauf erkannt werden.

Typisch ist, daß das Vorgemelk wegen der höchsten Anfälligkeit zur Verkeimung in den ersten Sekunden des Melkvorganges einen höheren Leitwert aufweist, der dann rasch abfällt und im Hauptgemelk bei einer gesunden Kuh einen niedrigen Wert annimmt, der sich kontinuierlich (stetig) verändern kann. Durch den Vergleich des Vorgemelks zwischen den einzelnen Zitzen und durch Vergleiche des laufenden Vorgemelks mit einem oder mehreren früheren Vorgemelken derselben Zitze kann mit hoher Sicherheit erkannt werden, ob auffällige Abweichungen vorhanden sind.

Gemäß einer Weiterbildung der Erfindung erfolgt eine weitere Filterung der Leitfähigkeitssignale dadurch, daß Amplitudenwerte, die über mehr als ein vorgegebenes Maß von früheren Amplitudenwerten derselben Meßzelle abweichen, unterdrückt werden. Damit wird sichergestellt, daß Ausreißer das Meßergebnis nicht verfälschen. Das vorgegebene Maß wird zweckmäßigerweise als Anteil oder Prozentsatzes der früheren Amplitudenwerte festgelegt, bei denen es sich vorzugsweise um die zuletzt gemessenen Amplitudenwerte derselben Meßzelle handelt.

Da die Erkennung on-line erfolgt, ist es mit dem erfindungsgemäßen Verfahren möglich, Milch, die von einer erkrankten Zitze geliefert wurde, vor Einführung in den Milchsammler auszusondern. Auf diese Weise wird sichergestellt, daß keimhaltige Milch nicht mit gesunder Milch vermischt wird.

Im folgenden wird unter Bezugnahme auf die Zeichnungen ein Ausführungsbeispiels der Erfindung näher erläutert.

Es zeigen:
- Fig. 1: eine schematische Darstellung des Milchflusses von einem Zitzenbecher bis zu einem Milchtank,
- Fig. 2: eine schematische Darstellung der Meßkammer, die die Leitfähigkeits-Meßzelle enthält,
- Fig. 3: ein Blockschaltbild der Auswerteeinrichtung,
- Fig. 4: charakteristische Zeitverläufe des Leitfähigkeitssignals über einen Melkvorgang,
- Fig. 5: die Gewinnung der charakteristischen Leitfähigskeitssignale und
- Fig. 6: den zeitlichen Verlauf der Leitfähigkeit bei einer Zitze, bei der über mehrere Tage hinweg eine fortschreitende Erkrankung festgestellt wird.

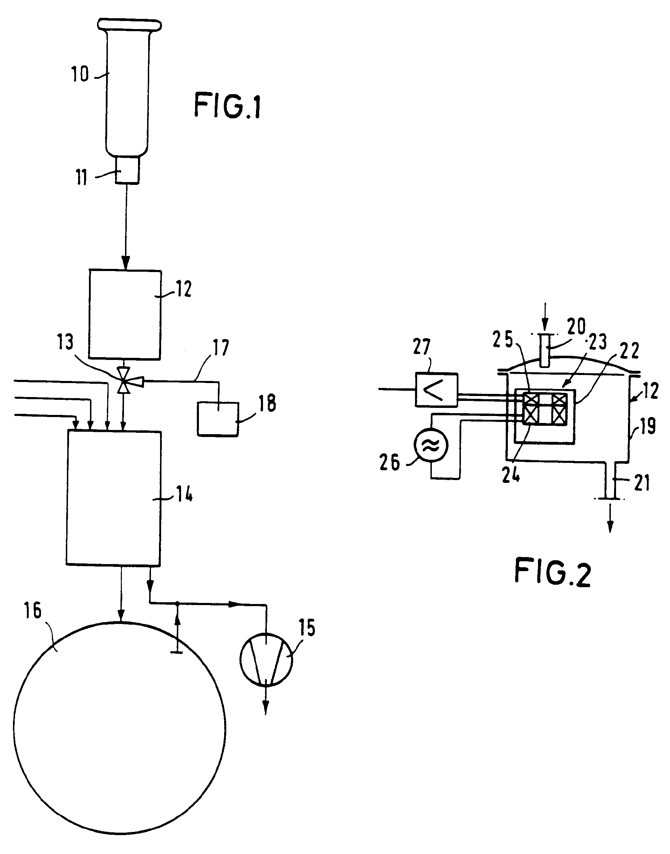

In Fig. 1 ist der Zitzenbecher 10 einer Melkmaschine dargestellt, die vier solcher Zitzenbecher aufweist. Der Zitzenbecher 10 wird in bekannter Weise an die Zitze einer Kuh angesetzt. Am Auslaß des Zitzenbechers 10 befindet sich ein Durchflußmesser 11 und von diesem führt eine Leitung in die Meßkammer 12. Von der Meßkammer 12 gelangt die Milch über ein magnetisch gesteuertes Ventil 13 in den Milchsammler 14. Im Milchsammler 14 vereinigen sich die Milchströme aller vier Zitzenbecher. Der Milchsammler ist an eine periodisch arbeitende Saugpumpe 15 sowie an einen Tank 16 angeschlossen, in dem die Milch gesammelt wird.

Von dem Ventil 13 führt eine Zweigleitung 17 in einen Sammler 18 für minderwertige Milch. Solange in der Meßkammer 12 gesunde Milch festgestellt wird, wird das Ventil 13 auf den Milchsammler 14 geschaltet, jedoch wird es auf den Sammler 18 umgeschaltet, wenn in der zugehörigen Meßkammer 12 anhand eines zu hohen Leitwertes keimhaltige Milch festgestellt wird.

Die Meßkammer 12 ist in Fig. 2 schematisch dargestellt. Sie weist ein Gehäuse 19 auf mit einem Einlaß 20 im Deckel und einem Auslaß 21 im Boden. Im Gehäuse 19 befindet sich unterhalb des Einlasses 20 ein Auffangbehälter 22, in den die Milch vom Einlaß 20 herabfällt. Im Auffangbehälter 22 ist die Meßzelle 23 angeordnet, die aus einer Erregerspule 24 und einer hierzu coaxialen Empfängerspule 25 oder Sekundärspule besteht. Die beiden Spulen 24 und 25 bilden einen eisenlosen Transformator. Die Erregerspule 24 wird von einem Oszillator 26 mit einer Erregerfrequenz von ca. 5 bis 10 kHz gespeist. In Abhängigkeit von dem Leitwert der im Inneren der Spulen befindlichen Milch entsteht an der Empfängerspule 25 eine Spannung, die von einem Verstärker 27 verstärkt wird. Diese Spannung bildet das Leitfähigkeitssignal.

Zur automatischen Identifizierung der Kuh ist diese mit einer entsprechenden maschinenlesbaren Codierung versehen. Dadurch wird erreicht, daß Leitfähigkeitswerte stets derselben Kuh zugeordnet und in einem für diese Kuh vorgesehenen Speicherbereich abgespeichert werden. Schließlich ist darauf zu achten, daß eine eindeutige Zuordnung zwischen den Zitzenbechern und den Zitzen der Kuh besteht, d.h. daß derselbe Zitzenbecher jedes Mal an dieselbe Zitze angesetzt wird.

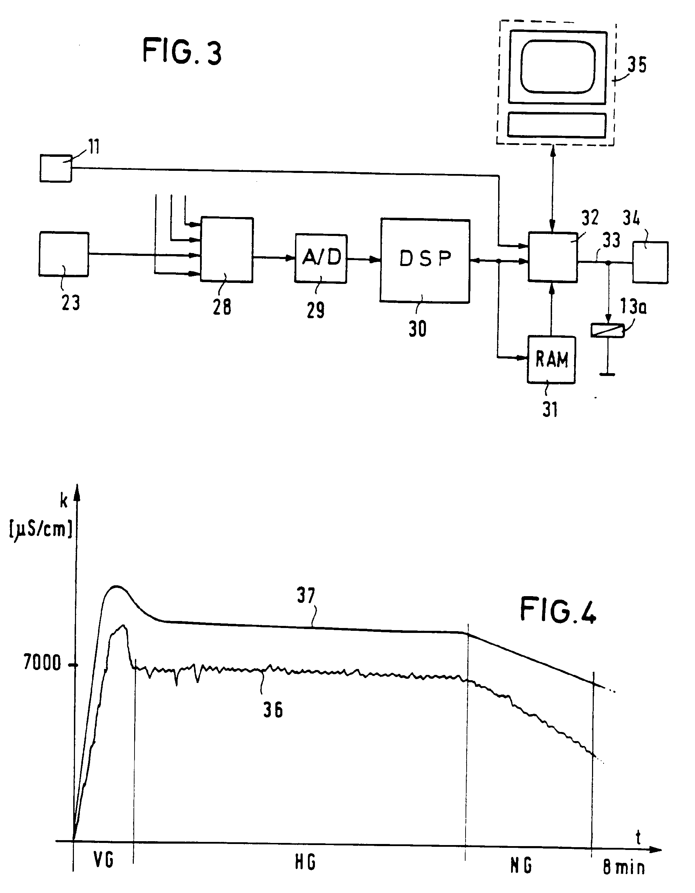

Gemäß Fig. 3 wird das von der Meßzelle 23 gelieferte Leitfähigkeitssignal einem Multiplexer 28 zugeführt, der außerdem die Leitfähigkeitssignale der den drei anderen Zitzenbechern zugeordneten Meßzellen empfängt und die vier Leitfähigkeitssignale im Zeit-Multiplex-Betrieb einem Analog/Digital-Umsetzer 29 zuführt. Die Leitfähigkeitssignale werden in dem digitalen Signalprozessor (DSP) 30 in digitaler Form verarbeitet. Der Signalprozessor führt eine Fast-Fourier-Analyse und eine Tiefpaßfilterung bzw. Bandfilterung durch, wobei Frequenzen zwischen 0,01 und 0,1 Hz durchgelassen und alle übrigen Frequenzen unterdrückt werden. Außerdem wird im Signalprozessor 30 eine Amplitudenfilterung vorgenommen, wobei diejenigen Amplitudenwerte, die über mehr als ein vorgegebenes Maß (15 %) von früheren Amplitudenwerten derselben Meßzelle nach unten abweichen, unterdrückt werden.

Die gefilterten Leitfähigkeitssignale, die repräsentative Ergebnisse für die noch zu erläuternden Intervalle eines Melkvorgangs darstellen, werden in einer Tabelle in dem RAM-Speicher 31 abgespeichert. Ferner werden die Ausgangssignale des Signalprozessors 30 einem weiteren Prozessor 32 zugeführt, in dem sie mit Signalen, die im Speicher 31 abgespeichert worden sind, verglichen werden, also mit Leitfähigkeitssignalen früherer Melkvorgänge.

Der Ausgang 33 des Prozessors 32 liefert bei Erkennung von Milch aus einer erkrankten Zitze ein Signal an eine Alarmvorrichtung 34 sowie an den Magneten 13a des Magnetventils 13 (Fig. 1), so daß dieses Ventil auf den Sammler 18 umgeschaltet wird.

An den Prozessor 32 ist ferner eine Ein-/Ausgabeeinrichtung 25 angeschlossen, an der die Leitwerte des laufenden Melkvorgangs graphisch auf einem Bildschirm angezeigt werden können, sowie ferner auch die aus dem Speicher 31 abgerufenen Leitwerte früherer Melkvogänge. Ferner enthält die Ausgabevorrichtung 35 eine Tastatur mit der die Grenzwerte und zulässigen Anteile (Prozentsätze) für die Filterung und Auswertung der Leitwertsignale eingestellt werden können.

Der Prozessor 32 empfängt ferner das Signal des Durchflußmessers 11, der die Menge der abgegebenen Milch mißt. Fließt Milch aus einer Zitze nur sehr spärlich, so wird ebenfalls ein Meldesignal erzeugt. Diese Funktion der Durchflußüberwachung kann auch den Melkvorgang beenden, wenn das Signal an die Saugpumpe 15 gegeben wird.

Der digitale Signalprozessor 30 bewirkt das Herausfiltern der verwertbaren Leitfähigkeitssignale sowie die Aufbereitung dieser Signale zu einem charakteristischen Signal für jedes Intervall. Der Prozessor 32 führt dagegen die Verwaltung und Auswertung der aufbereiteten charakteristischen Leitfähigkeitswerte durch.

In Fig. 4 ist der zeitliche Verlauf eines typischen Melkvorgangs dargestellt, wobei auf der Ordinate der spezifische Leitwert k in µS/cm aufgetragen ist. Unter "Leitwert" ist stets der spezifische elektrische Leitwert zu verstehen.

Der Melkvorgang besteht aus den Phasen Vorgemelk VG, Hauptgemelk HG und Nachgemelk NG. Mit 36 ist der typische zeitliche Verlauf des Leitwerts von Milch aus einer gesunden Zitze bezeichnet. Man erkennt, daß der Leitwert starken Schwankungen unterworfen und "verrauscht" ist, was insbesondere auf den Einfluß der Saugstöße der Saugpumpe 15 (Fig. 1) zurückzuführen ist, die in der Meßkammer wirksam sind.

Mit 37 ist der geglättete Verlauf der Leitwertkurve von Milch aus einer erkrankten Zitze bezeichnet. Die Leitwerte liegen um etwa 1000 µS/cm über der Kurve 36.

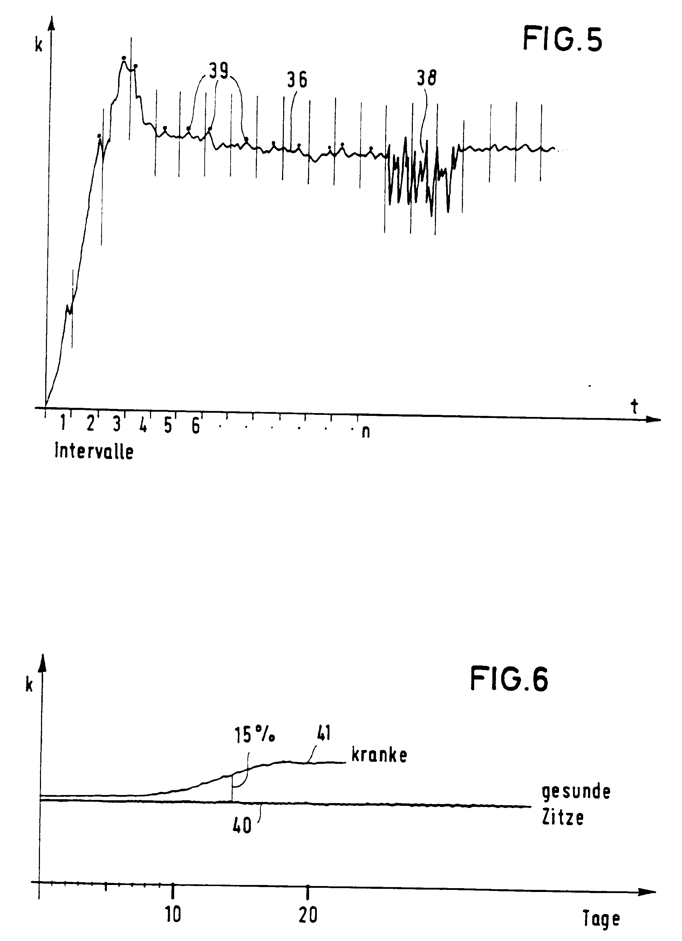

Anhand von Fig. 5 wird die Gewinnung der für die Auswertung bestimmten charakteristischen Leitfähigkeitswerte erläutert.

Der gesamte Melkvorgang ist in zahlreiche Intervalle von jeweils 6 Sekunden Dauer aufgeteilt, die in Fig. 5 entlang der Zeitachse t aufgetragen sind und die laufend durchnumeriert sind. In jedem dieser Intervalle wird ein charakteristischer Leitfähigkeitswert bestimmt, der durch einen Punkt markiert ist. Der charakteristische Leitfähigkeitswert ist bei dem dargestellten Ausführungsbeispiel der Spitzenwert in dem jeweiligen Intervall, also die Amplitude einer Signalspitze.

Diejenigen Frequenzen, die außerhalb des Durchlaßbereichs des digitalen Frequenzfilters liegen, werden unterdrückt. Beispielsweise enthält die Kurve 36 in Fig. 5 einen ausgeprägten Bereich 38 von hoher Frequenz und großer Amplitudenschwankung. Dieser Bereich 38 wird unterdrückt. In ihm erfolgt keine Bildung eines zur Auswertung bestimmten charakteristischen Leitfähigkeitswerts. Durch die Filterung wird keine Glättung des unerwünschten Signalbereichs vorgenommen, sondern eine Nicht-Berücksichtigung bei der Auswertung.

Der digitale Signalprozessor 30 liefert die charakteristischen Leitfähigkeitswerte, die in Fig. 5 durch Punkte markiert und mit 39 bezeichnet sind, zusammen mit den zugehörigen Intervallnummern an den Prozessor 32 und den Speicher 31. Im Speicher 31 wird der gesamte Melkvorgang, getrennt nach Zitzenbechern, dokumentiert, wobei in einer Tabelle für jeden Zitzenbecher alle charakteristischen Leitfähigkeitswerte 39 mit ihren Intervallnummern abgespeichert werden.

Der Prozessor 32 vergleicht die charakteristischen Leitfähigkeitswerte der vier Zitzenbecher, Intervall für Intervall, untereinander, um eine von den übrigen Kurven abweichende Kurve einer Zitze feststellen zu können, und er vergleicht auch die Kurven des aktuellen Melkvorgangs mit den gespeicherten Kurven früherer Melkvorgänge, nach Zitzen getrennt, wobei die Intervalle einander zugeordnet sind.

In Fig. 6 ist der Leitwert k über der Zeit aufgetragen, wobei die Zeit in Tagen angegeben ist. Mit 40 ist die Kurve einer gesunden Zitze und mit 41 die Kurve einer erkrankten Zitze bezeichnet. Jede der Kurven 40 und 41 besteht aus Punkten, von denen jeder einen charakteristischen Wert eines Melkvorgangs darstellt, beispielsweise den Mittelwert sämtlicher charakteristischer Werte 39. Die Erkrankung der Zitze wird dann festgestellt, wenn der Wert der Kurve 41 um einen bestimmten Prozentsatz (15 %) von den früheren Werten derselben Zitze abweicht. Hierbei befindet sich die erkrankte Zitze noch im Frühstadium der Erkrankung.

## Patentansprüche

1. Verfahren zur Überwachung der Qualität bei der Milchabgabe von Tieren, bei welchem den Zitzenbechern einer Melkvorrichtung Leitfähigkeitsmeßzellen zugeordnet werden, deren Leitfähigkeitssignale in einer Auswerteeinrichtung ausgewertet werden, in dem die Leitfähigkeitssignale der Meßzellen untereinander verglichen werden, wobei eine Abweichung der Signale einer Meßzelle von denen der übrigen Meßzellen erkannt wird,
dadurch gekennzeichnet,
daß
a) die Leitfähigkeitssignale in digitale Form umgewandelt und in einem digitalen Signalprozessor (30) gefiltert werden, der Signalfrequenzen über 0,2 Hz unterdrückt;
b) der Melkvorgang für jeden Zitzenbecher (10) in Zeitintervalle aufgeteilt wird;
c) für jedes Zeitintervall ein charakteristischer Leitfähigkeitswert (39) ermittelt und gespeichert wird und
d) die Leitfähigkeitswerte derjenigen Zeitintervalle miteinander verglichen werden, die vom Beginn der Melkvorgänge der einzelnen Zitzenbecher denselben zeitlichen Abstand haben.

2. Verfahren nach Anspruch 1,
dadurch gekennzeichnet,
daß eine weitere Filterung dadurch erfolgt, daß Amplitudenwerte, die über mehr als ein vorgegebenes Maß von früheren Amplitudenwerten derselben Meßzelle bei demselben Melkvorgang abweichen, unterdrückt werden.

3. Verfahren nach Anspruch 2,
dadurch gekennzeichnet,
daß bei mindestens einem Melkvorgang die charakteristischen Leitfähigkeitswerte (39) der Zeitintervalle nach Zitzenbechern getrennt in einem Speicher (31) abgespeichert und bei einem nachfolgenden Melkvorgang die neuen Leitfähigkeitswerte mit den gespeicherten Werten aus den entsprechenden Zeitintervallen verglichen werden, wobei Unterschiede, die ein vorgegebenes Maß unterschreiten, erkannt werden.

4. Verfahren nach einem der Ansprüche 1 bis 3,
dadurch gekennzeichnet,
daß der Signalprozessor (30) einen Durchlaßbereich zwischen 0,01 und etwa 0,1 Hz hat.

5. Verfahren nach einem der Ansprüche 1 bis 4,
dadurch gekennzeichnet,
daß die Mittelwerte von charakteristischen Leitfähigkeitswerten (39) nach Zitzenbechern getrennt bei mindestens einem Melkvorgang gebildet und mit entsprechenden Mittelwerten nachfolgender Melkvorgänge verglichen werden.

6. Verfahren nach einem der Ansprüche 1 bis 5,
dadurch gekennzeichnet,
daß der charakteristische Leitfähigkeitswert (39) eines Zeitintervalls der Spitzenwert des gefilterten Leitfähigkeitssignals ist.

7. Verfahren nach einem der Ansprüche 1 bis 6,
dadurch gekennzeichnet,
daß die Leitfähigkeitssignale aller Meßzellen (23) im Zeitmultiplexbetrieb verarbeitet werden.

8. Verfahren nach einem der Ansprüche 1 bis 7,
dadurch gekennzeichnet,
daß Milch aus einem Zitzenbecher (10), dessen zugeordnete Meßzelle (23) ein abnormes Leitfähigkeitssignal liefert, aus dem Milchfluß abgezweigt wird.

## Claims

1. Method, for monitoring the quality during the milking of animals, in which the teat cups of a milking device are associated with conductivity-measuring cells, the conductivity signal, of which are evaluated in an evaluating equipment, in which the conductivity signals of the measuring cells are compared one among the other, wherein a deviation of the signals of one measuring cell from those of the remaining measuring cells is recognised, characterised thereby, that
a) the conductivity signals are converted into digital form and filtered in a digital signal processor (30), which suppresses signal frequencies above 0.2 hertz,
b) the milking operation is divided into time intervals for each teat cup (10),
c) a characteristic conductivity value (39) is ascertained and stored for each time interval and
d) the conductivity values of those time intervals are compared one with the other, which have the same time spacing from the beginning of the milking operations of the individual teat cups.

2. Method according to claim 1, characterised thereby, that a further filtering takes place thereby, that amplitude values, which deviate by more than a preset amount from earlier amplitude values of the same measuring cell during the same milking operation, are suppressed.

3. Method according to claim 2, characterised thereby, that the characteristic conductivity values (39) of the time intervals are stored in a storage device (31) separately according to teat cups during at least one milking operation and the new conductivity values during a milking operation are compared with the stored values from the corresponding time intervals, wherein differences, which fall below a preset amount, are recognised.

4. Method according to one of the claims 1 to 3, characterised thereby, that the signal processor (30) has a transmission range between 0.01 and about 0.1 hertz.

5. Method according to one of the claims 1 to 4, characterised thereby, that the mean values of characteristic conductivity values (39) are formed during at least one milking operation separately according to teat cups and compared with corresponding mean values of subsequent milking operations.

6. Method according to one of the claims 1 to 5, characterised thereby, that the characteristic conductivity value (39) of a time interval is the peak value of the filtered conductivity signal.

7. Method according to one of the claims 1 to 6, characterised thereby, that the conductivity signals of all measuring cells (23) are processed in time multiplex operation.

8. Method according to one of the claims 1 to 7, characterised thereby, that milk from a teat cup (10), the associated measuring cell (23) of which supplies an abnormal conductivity signal, is branched off from the milk flow.

## Revendications

1. Procédé, permettant de contrôler la qualité du lait lors de la traite d'animaux, qui consiste à utiliser des cellules de mesure de conductivité associées aux gobelets trayeurs d'un dispositif de traite, à analyser les signaux de conductivité provenant de ces cellules de mesure dans un dispositif analyseur dans lequel ils sont comparés entre eux, et à détecter un écart qui survient entre les signaux d'une cellule de mesure et ceux des autres cellules de mesure ce procédé étant caractérisé par le fait que:
**a)** les signaux de conductivité sont convertis sous forme numérique et filtrés dans un processeur (30) de signaux numériques qui supprime les fréquences de signaux supérieures à 0,2 Hz;
**b)** le processus de la traite est divisé en périodes pour chaque gobelet trayeur (10);
**c)** pour chaque période, une valeur caractéristique (39) de conductivité est calculée et mise en mémoire et
**d)** les valeurs de conductivité des périodes, qui ont le même intervalle de temps depuis le début des processus de traite des différents gobelets trayeurs, sont comparées entre elles.

2. Procédé selon la revendication 1, caractérisé par le fait qu'un autre filtrage a lieu en supprimant les valeurs des amplitudes qui diffèrent des valeurs des amplitudes antérieures de la même cellule de mesure pendant le même processus de traite et les dépassent dans une mesure supérieure à une mesure prédéfinie.

3. Procédé selon la revendication 2, caractérisé par le fait qu'au cours d'au moins un processus de traite, les valeurs caractéristiques (39) de conductivité des périodes sont stockées séparément dans une mémoire (31), suivant les gobelets trayeurs, et que, lors d'un processus de traite suivant de près les nouvelles valeurs de conductivité sont comparées aux valeurs mises en mémoire issues des périodes correspondantes, des différences dépassant une mesure prédéfinie étant détectées.

4. Procédé selon l'une quelconque des revendications 1 à 3, caractérisé par le fait que le processeur (30) de signaux a une bande passante comprise entre 0,01 et environ 0,1 Hz.

5. Procédé selon l'une quelconque des revendications 1 à 4, caractérisé par le fait que les moyennes des valeurs caractéristiques (39) de conductivité, suivant les gobelets trayeurs, sont formées séparément au cours d'au moins un processus de traite et sont comparées aux moyennes correspondantes des processus de traite qui les suivent.

6. Procédé selon l'une quelconque des revendications 1 à 5, caractérisé par le fait que la valeur caractéristique (39) de conductivité d'une période est la valeur de crête du signal de conductivité filtré.

7. Procédé selon l'une quelconque des revendications 1 à 6, caractérisé par le fait que les signaux de conductivité de toutes les cellules de mesure (23) sont traités dans une opération de multiplexage dans le temps.

8. Procédé selon l'une quelconque des revendications 1 à 7, caractérisé par le fait que le lait provenant d'un gobelet trayeur (10) dont la cellule de mesure (23) associée livre un signal de conductivité anormal est séparé du lait qui s'écoule.
